# EUROPÄISCHE PATENTSCHRIFT

(11) **EP 2 788 307 B1**
(45) Veröffentlichungstag und Bekanntmachung des Hinweises auf die Patenterteilung: **03.07.2019**
(21) Anmeldenummer: 12794636.6
(22) Anmeldetag: 15.11.2012
(51) Int. Cl.: C07C 17/25, C07C 17/383, C07C 19/045, C07C 21/06, B01D 3/00, B01D 3/14

(54) **VERFAHREN ZUR WÄRMERÜCKGEWINNUNG IN VINYLCHLORID-MONOMERANLAGEN ODER IM ANLAGENVERBUND VINYLCHLORID / POLYVINYLCHLORID UND DAFÜR GEEIGNETE VORRICHTUNG**
PROCESS AND APPARATUS FOR HEAT RECOVERY IN VINYL CHLORIDE MONOMER PLANTS OR IN INTEGRATED VINYL CHLORIDE MONOMER/POLYVINYL CHLORIDE PLANTS
PROCÉDÉ DE RÉCUPÉRATION DE CHALEUR DANS DES INSTALLATIONS DE CHLORURE DE VINYLE MONOMÈRE OU DANS UN ENSEMBLE D'INSTALLATIONS DE CHLORURE DE VINYLE/CHLORURE DE POLYVINYLE, ET DISPOSITIF APPROPRIÉ CORRESPONDANT

(30) Priorität: 08.12.2011 DE 102011120479; 13.04.2012 DE 102012007339
(43) Veröffentlichungstag der Anmeldung: 15.10.2014
(73) Patentinhaber: thyssenkrupp Industrial Solutions AG, 45143 Essen (DE); Vinnolit GmbH & Co. KG, 84508 Burgkirchen (DE)
(72) Erfinder: BENJE, Michael, 65812 Bad Soden (DE); KAMMERHOFER, Peter, 84508 Burgkirchen (DE)
(74) Vertreter: Kutzenberger Wolff & Partner
(86) Internationale Anmeldenummer: PCT/EP2012/004747
(87) Internationale Veröffentlichungsnummer: WO 2013/083230

(56) Entgegenhaltungen:
- EP-A1- 0 131 932
- DE-A1- 3 519 161
- Alan Rossiter: "Energy Management", Kirk-Othmer Encyclopedia of Chemical Technology, Vol. 10, 15 October 2004 (2004-10-15), pages 133-168, XP055071650, DOI: 10.1002/0471238961.0514051819200509.a01.pu b2 Retrieved from the Internet: URL:http://onlinelibrary.wiley.com/store/1 0.1002/0471238961.0514051819200509.a01.pub 2/asset/enerstei.a01.pdf?v=1&t=hj8747wq&s= 540024cc7811c119baa6374a036dbf4f7c345ee7 [retrieved on 2013-07-17]

## Beschreibung

Die Erfindung betrifft ein Verfahren sowie eine Vorrichtung zur Herstellung von 1,2-Dichlorethan (nachstehend "DCE") und zielt ab auf den Betrieb einer Kolonne zur Destillation von 1,2-Dichlorethan bei Temperaturen die einerseits ausreichend hoch sind, um durch Kondensation zumindest eines Teils des Kopfstroms (Brüdens) der Kolonne nutzbare Wärme zu erzeugen, die aber anderseits noch nicht zur Schädigung des destillierten DCE durch thermische Zersetzung führen.

Speziell zielt die Erfindung auf die Beheizung von Wärmesenken in einer Anlage zur Herstellung von Vinylchlorid oder in einem Anlagenverbund zur Herstellung von Vinylchlorid (nachstehend "VCM") und Polyvinylchlorid (nachstehend "PVC") mittels der am Kopf einer Destillationskolonne rückgewonnenen Wärmeenergie.

DCE wird überwiegend als Zwischenprodukt der Herstellung von monomerem Vinylchlorid verwendet, welches wiederum als Ausgangsstoff für die Herstellung von Polyvinylchlorid eingesetzt wird. Bei der Umsetzung von DCE zu monomerem Vinylchlorid entsteht Chlorwasserstoff HCl. Dieser wird bevorzugt bei der Herstellung von DCE durch Oxychlorierung von Ethen mittels HCl und Sauerstoff eingesetzt. Ein alternativer Herstellungsweg von DCE führt über die Direktchlorierung von Ethen mittels Chlor. Bei der großtechnischen Herstellung von DCE werden beide Wege beschritten, so dass hinsichtlich des erzeugten und verbrauchten Chlorwasserstoffs eine ausgewogene Bilanz entsprechend den folgenden Reaktionsgleichungen erreicht wird:

Cl₂ + C₂H₄ → C₂H₄Cl₂ + 218 kJ/Mol

C₂H₄Cl₂ → C₂H₃Cl + HCl - 71 kJ/Mol

C₂H₄ + 2 HCl + ½ O₂ → C₂H₄Cl₂ + H₂O + 238 kJ/Mol

Ein Anlagenkomplex zur Produktion von Vinylchlorid (nachstehend "VCM-Komplex" genannt) besteht im Wesentlichen aus:
- einer Anlage zur Herstellung von 1,2-Dichlorethan (DCE) aus Ethen und Chlor ("Direktchlorierung", optionaler Anlagenteil); und
- einer Anlage zur Herstellung von 1,2-Dichlorethan aus Ethen, Chlorwasserstoff und Sauerstoff ("Oxichlorierung"); und
- einer Anlage zur destillativen Reinigung von 1,2-Dichlorethan (Herstellung von "Feed-DCE"), und
- einer Anlage zur thermischen Spaltung des destillativ gereinigten "Feed-DCE" zu Vinylchlorid und Chlorwasserstoff; und
- einer Anlage zur destillativen Abtrennung des Chlorwasserstoffs und nicht umgesetzten 1,2-Dichlorethans sowie zur Reinigung des Vinylchlorids.

Der durch thermische Spaltung des 1,2-Dichlorethans gewonnene Chlorwasserstoff wird in die Oxichlorierungsanlage zurückgeführt und dort mit Ethen und Sauerstoff wieder zu DCE umgesetzt.

Die Reaktionsschritte der Direktchlorierung und der Oxichlorierung sind stark exotherm während die thermische Spaltung von DCE zu VCM und Chlorwasserstoff endotherm ist.

Der oben beschriebene VCM-Komplex kann in der sogenannten ausgewogenen oder "balanced" Betriebsweise betrieben werden, in der das gesamte in der Anlage erzeugte DCE auch in der VCM-Teilanlage weiter verarbeitet wird bzw. bei der kein Import von DCE erforderlich ist.

Neben der vorstehend genannten ausgewogenen Betriebsweise existieren auch Betriebsarten bzw. Anlagen zur Herstellung von DCE, bei denen die DCE-Menge, die in der ausgewogenen Betriebsweise in der Direktchlorierung produziert würde ganz oder teilweise durch importiertes DCE (Import-DCE) ersetzt wird. Diese Betriebsart oder Anlagenkonfiguration nennt der Fachmann die unausgewogene oder "unbalanced" Betriebsweise.

Eine weitere unausgewogene Betriebsweise besteht darin, dass in der Teilanlage zur Herstellung von DCE mehr DCE erzeugt wird als in der thermischen Spaltung zu VCM verbraucht wird. Dieses überschüssige DCE wird nach destillativer Reinigung als sogenanntes "sales DCE" verkauft. In der "sales-DCE"-Betriebsweise werden in der Regel mehr Kolonnen zur Aufarbeitung des DCE eingesetzt, als bei den anderen Betriebsweisen. Diese zusätzlichen Kolonnen stellen zusätzliche Wärmesenken dar und können mit aus anderen Anlagenteilen stammender Wärme betrieben werden.

Aus dem Stand der Technik sind zahlreiche Massnahmen zur Energieeinsparung bzw. Wärmerückgewinnung in Anlagen zur Herstellung von VCM und PVC bekannt. Solche Massnahmen führen zu einer deutlichen Senkung der Betriebskosten und tragen damit ganz wesentlich zur Wirtschaftlichkeit der Anlage bei. Ebenso tragen solche Massnahmen auch wesentlich zur Verringerung des CO₂-Ausstoßes der Anlage bei.

Hierzu zählen solche Massnahmen, welche die Reaktionswärme der exothermen Reaktionsschritte nutzen, um Wärmesenken im Prozess zu beheizen. So wird beispielsweise mit der Reaktionswärme der Oxichlorierung Dampf erzeugt, mit dem z.B. Edukt-Vorwärmer oder Destillationskolonnen beheizt werden können.

Aufgrund des relativ hohen Temperaturniveaus der Oxichlorierungsreaktion ist der erzeugte Dampf zur Beheizung der meisten Wärmesenken im Prozess geeignet. Bevorzugt werden mit diesem Dampf natürlich Wärmesenken beheizt, die ihrerseits ein relativ hohes Temperaturniveau erfordern.

Die in der Teilanlage der Oxichlorierung erzeugte Dampfmenge ist nicht ausreichend, um alle Wärmesenken in einem Anlagenkomplex zur Herstellung von VCM zu beheizen. So wurde nach weiteren Möglichkeiten zur Wärmerückgewinnung bzw. Energieeinsparung gesucht.

Eine Möglichkeit hierzu ist die Nutzung der Reaktionswärme der Direktchlorierung, die auf einem niedrigeren Temperaturniveau anfällt als die der Oxichlorierung. Hierzu existiert in der Literatur eine Vielzahl von Vorschlägen.

So wird z.B. in der DE 32 25 732 A1 vorgeschlagen, mit einem Zirkulationsstrom des flüssigen Reaktionsmediums aus der Direktchlorierung eine Destillationskolonne zu beheizen.

In der DE 31 37 513 A1 wird vorgeschlagen, die Reaktionswärme für Heizzwecke oder zur Dampferzeugung zu verwenden. Eine Einschränkung bei der Dampferzeugung mittels der Reaktionswärme der Direktchlorierung besteht allerdings darin, dass die Reaktionstemperatur hierfür auf einen Wert angehoben werden muss, der schon stark die Bildung von Nebenprodukten begünstigt, was wiederum die Wirtschaftlichkeit des Verfahrens beeinträchtigt. Ein Ausweg bestünde darin, dampfförmiges Reaktions-medium aus dem Direktchlorierungsreaktor mechanisch zu komprimieren und dann für Heizwecke einzusetzen, wie in der WO 01/21564 A1 vorgeschlagen wurde. Nachteilig hierbei sind die Investitionskosten für den erforderlichen Verdichter sowie die Energiekosten für die Verdichtung.

Ebenso existieren Vorschläge zur Beheizung von Kolonnen mit dampfförmigem Reaktionsmedium, wie z.B. in der DE 199 16 753 C1 und in der WO 98/01407 A1 beschrieben, sowie gleichzeitig mit dampfförmigem und flüssigem Reaktionsmedium, wie in der DE 199 53 762 A1 beschrieben.

Da Direktchlorierungsanfagen bzw. Anlagenkomplexe zur Herstellung von Vinylchlorid und Polyvinylchlorid oft im Verbund mit einer Anlage zur Chlor-Alkali-Elektrolyse betrieben werden, wurde auch vorgeschlagen, die Reaktionswärme der Direktchlorierung zur Konzentration von wässriger Natronlauge zu nutzen, wie z.B. in der DE10 2005 044 177 A1 beschrieben.

Auch innerhalb der Teilanlage zur destillativen Reinigung des 1,2-Dichlorethan bestehen Möglichkeiten zur Energieeinsparung. Im Allgemeinen besteht die Teilanlage in einem VCM-Komplex aus einer sogenannten Entwässerungskolonne, in der aus dem DCE Wasser sowie Leichtsieder abgetrennt werden. In Abhängigkeit von der Anlagenkonfiguration können in der Anlage ein oder mehrere weitere Kolonnen eingesetzt werden, z.B. zur Abtrennung von Leichtsiedern. Der Sumpfstrom der Entwässerungskolonne wird in der Regel in einer sogenannten Hochsiederkolonne oder DCE-Kolonne weiter aufgereinigt. Weiterhin wird in die Hochsiederkolonne DCE eingespeist, das aus dem Produktgemisch der thermischen DCE-Spaltung abgetrennt wurde (sog. Rück-DCE). In der Hochsiederkolonne werden höher als DCE siedende Substanzen abgetrennt. Das Kopfprodukt der Hochsiederkolonne ist das "Feed-DCE", das in der thermischen DCE-Spaltung eingesetzt wird. Der Sumpfstrom der Hochsiederkolonne wird meist in einer mit Unterdruck betriebenen sogenannten Vakuumkolonne weiter aufkonzentriert. Das in der Vakuumkolonne abgetrennte DCE wird dem Feed-DCE-Strom vom Kopf der Hochsiederkolonne zugemischt. Die abgetrennten Hochsieder werden einer Aufarbeitung zugeführt.

Der grösste Energieverbraucher innerhalb der destillativen DCE-Reinigung ist die Hochsiederkolonne. Prinzipiell ist die in der Direktchlorierungsanlage rückgewinnbare Wärmemenge nicht hoch genug, um den Gesamtenergiebedarf dieser Kolonne zu decken. Die fehlende Wärmemenge muss durch Beheizung mit Dampf zugeführt werden. Weiterhin ist die bei einer Beheizung der Hochsiederkolonne mit der Reaktionswärme der Direktchlorierung erreichbare Brüdentemperatur der Hochsiederkolonne nicht hoch genug, um aus dem Brüden wirtschaftlich Wärme zurück zu gewinnen.

Die vorliegende Erfindung richtet sich auf ein Verfahren zur Wärmerückgewinnung an der oder den Hochsiederkolonnen (oft auch "DCE-Kolonne" genannt) der Teilanlage zur destillativen Reinigung von DCE in einem VCE-Komplex.

Hier wurde in der DE 34 40 685 A1 schon vorgeschlagen, den Brüden vom Kopf dieser Kolonne mechanisch zu verdichten und zum Beheizen derselben Kolonne zu verwenden. Energetisch günstiger ist es jedoch, die Hochsiederkolonne bei einem Druck bzw. einer Temperatur zu betreiben die hoch genug ist, dass der Kopfstrom (Brüden) der Kolonne zur Ausführung von Wärmerückgewinnungsmassnahmen geeignet ist. Anderseits darf die Kopftemperatur der Kolonne nicht so hoch sein, dass das Produkt (Feed-DCE) durch Zersetzung geschädigt wird.

DE 35 19 161 A1 beschreibt ein Verfahren zur Reinigung von DCE, bei dem eine Destillationskolonne so betrieben wird, dass eine Kopftemperatur von 125-180 °C resultiert. Das am Kopf dieser Kolonne abgeführte gasförmige DCE wird durch Wärmetauscher geführt, die zur Erwärmung von DCE-enthaltenden Produktströmen dienen. Das in den Wärmetauschern kondensierte DCE wird sodann in die Kolonne rückgeführt und wird teilweise als gereinigtes Produkt abgeführt und weiterverwendet. Das beschriebene Verfahren erhöht die Energieeffizienz der Anlage erheblich. Gleichwohl kann nicht die gesamte im Kopfprodukt enthaltene thermische Energie genutzt werden, sondern der in den Wärmetauschern kondensierte DCE-Strom muss aktiv gekühlt werden. Es wäre wünschenswert, wenn auch der bislang nicht genutzte Wärmeinhalt des Kopfproduktes der Hochsiederkolonne zum Beheizen von Anlagenteilen genutzt werden könnte.

Johann Stichlmair et al., Chem. Eng. Technol. 12, 1989, Seiten 163-169, offenbart energiesparende Destillationsverfahren.

EP 0 131 932 B1 offenbart Verfahren zur Destillation von 1,2-Dichloroethan.

Überraschenderweise zeigte sich, dass die Hochsiederkolonne bei Kopftemperaturen zwischen ca. 120 -150 °C, vorzugsweise zwischen 127 und 135°C betrieben werden kann, ohne dass eine Schädigung des Produktes beobachtet wird. Dazu wird die Hochsiederkolonne unter Überdruck betrieben, z.B. im Bereich von 2,7 bis 5,3 bara, und die so erzeugten Brüden werden zur Gewinnung von Niederdruck-Wasserdampf eingesetzt, weicher für die indirekte Beheizung von Anlagenteilen der DCE-Anlage oder von Anlagenteilen der nachgeschalteten VCM-Anlage bzw. PVC-Anlage genutzt wird.

Bei der indirekten Beheizung von Anlagenteilen der DCE-Anlage, der VCM-Anlage und/oder der PVC-Anlage hat sich herausgestellt, dass dabei der gesamte nutzbare Wärmeinhalt der Brüden der Hochsiederkolonne(n) genutzt werden kann, indem Niederdruck-Wasserdampf erzeugt wird. Die Erzeugung von Niederdruck-Wasserdampf ist zur Beheizung räumlich weiter entfernter Wärmesenken darüber hinaus sicherheitstechnisch zu bevorzugen. Die Erzeugung von Niederdruck-Wasserdampf aus den Brüden einer unter Überdruck betriebenen Hochsiederkolonne einer DCE-Anlage ist bislang noch nicht beschrieben worden.

Die vorliegende Erfindung betrifft ein Verfahren zur Herstellung von Vinylchlorid durch thermische Spaltung von 1,2- Dichlorethan in einem Vinylchlorid-Komplex mit einer darin enthaltenen destillativen Reinigung von 1,2-Dichlorethan umfassend mindestens eine Hochsiederkolonne, in der höher als 1,2-Dichlorethan siedende Substanzen abgetrennt werden und einer gegebenenfalls daran angeschlossenen Polyvinylchlorid-Teilanlage mit den Maßnahmen
a) Betrieb der Hochsiederkolonne bei Kopftemperaturen zwischen 120 - 150 °C, und
b) Verwendung zumindestens eines Teils des Kopfstromes der Hochsiederkolonne zur Gewinnung von thermischer Energie, die in Wärmesenken einer nachgeschalteten Teilanlage zur Erzeugung von Vinylchlorid und/oder in Wärmesenken einer nachgeschalteten Teilanlage zur Herstellung von Polyvinylchlorid genutzt wird, wobei
c) der Kopfstrom zu einer indirekten Beheizung von Wärmesenken verwendet wird, indem zumindest ein Teil des Kopfstromes der Hochsiederkolonne in einem Wärmetauscher zur Erzeugung von Niederdruck-Wasserdampf genutzt wird und der Kopfstrom nach Kondensation und gegebenenfalls Unterkühlung in die Hochsiederkolonne rückgeführt wird und der Niederdruck-Wasserdampf zum Beheizen von ausgewählten Anlagenteilen eingesetzt wird,
   dadurch gekennzeichnet, dass die Anlagenteile ausgewählt sind aus
   - folgendem Anlagenteil aus dem Vinylchlorid-Komplex: einer Strippkolonne zur Reinigung oder Entfernung von Chlorwasserstoff aus dem Vinylchlorid; und/oder
   - folgenden Anlagenteilen aus der Polyvinylchlorid-Teilanlage: Vorrichtungen zur Entfernung von Rest-Monomer aus PVC, einer Vorentgasungseinrichtung und einer nachgeschalteten Entgasungskolonne, einer Strippkolonne zur Entfernung von Vinylchloridmonomer aus Abwasser, einer Vorrichtung zur Trocknung von PVC-Pulver und/oder einer Vorrichtung zur Erwärmung von Chargierwasser für die Polymerisationsreaktion.

Unter Niederdruck-Wasserdampf ist im Rahmen dieser beschreibung ein Wasserdampf zu verstehen, der typischerweise eine Temperatur im Bereich zwischen 115 und 145°C, vorzugsweise zwischen 118 und 130°C aufweist.

Der Kopfstrom wird zu einer indirekten Beheizung von Wärmesenken verwendet, indem zumindest ein Teil des Kopfstromes der Hochsiederkolonne zur Erzeugung von Niederdruck-Wasserdampf genutzt wird, beispielsweise in einem Wärmetauscher, wie einem Verdampfer, der Kopfstrom nach Kondensation und gegebenenfalls Unterkühlung in die Hochsiederkolonne rückgeführt wird und der Niederdruck-Wasserdampf zum Beheizen von ausgewählten Anlagenteilen eingesetzt wird. Diese Methode eignet sich vorzugsweise zum Beheizen von Anlagenteilen in weiterer Entfernung der Hochsiederkolonne, wie die Wärmesenken in einer nachgeschalteten VCM-Anlage und/oder einer nachgeschalteten PVC-Anlage.

Als Wärmetauscher für die indirekte Beheizung von Wärmesenken lassen sich alle gängigen Typen von Wärmetauschern einsetzen. Besonders bevorzugt sind Wärmetauschertypen, die eine Übertragung von Wärme bei besonders niedrigen Temperaturdifferenzen zwischen heißer und kalter Seite ermöglichen. Ganz besonders bevorzugt sind dabei Fallstromverdampfer, Plattenwärmetauscher, Spiralwärmetauscher oder Rohrbündelwärmetauscher, wobei letztere bevorzugt mit speziell für einen Wärmetausch bei niedrigen Temperaturdifferenzen geeigneten Rohren ausgerüstet sind (z.B. "high-flux"-Rohre der Fa. Honeywell UOP, Houston TX, USA).

Geeignete und bevorzugte Wärmesenken in einem Anlagenkomplex zur VCM / PVC-Herstellung sind:
Im VCM - Komplex:
   - Strippkolonne zur Reinigung (Entfernung von HCl) von Vinylchlorid.
In der PVC-Anlage:
   - Vorrichtungen zur Entfernung von Rest-Monomer (VCM) aus PVC, speziell eine Vorentgasungseinrichtung und eine nachgeschaltete Entgasungskolonne;
   - Strippkolonne zur Entfernung von VCM aus Abwasser;
   - Vorrichtung zur Trocknung von PVC-Pulver; und
   - Vorrichtung zur Erwärmung von Chargierwasser für die Polymerisationsreaktion.

Das erfindungsgemäße Verfahren ist dadurch gekennzeichnet, dass die indirekte Beheizung von Wärmesenken mit Niederdruck-Wasserdampf erfolgt, der aus dem Kopfstrom der Hochsiederkolonne der DCE-Anlage erzeugt worden ist.

Bevorzugt wird ein Verfahren zur Herstellung von Vinylchlorid und Polyvinylchlorid, bei dem das Sumpfprodukt der Hochsiederkolonne einen DCE Anteil von 90 - 97 Gew. % hat.

In einer bevorzugten Verfahrensvariante wird das destillativ in der Hochsiederkolonne gereingte DCE ohne weitere Behandlung zur thermischen Spaltung zu Vinylchlorid eingesetzt.

Der Betrieb der Hochsiederkolonne sowie der angeschlossenen Wärmetauscher kann überraschenderweise ohne Unterbrechung für lange Zeit erfolgen. So ist ein ununterbrochener Betrieb zwischen 6 und 24 Monaten durchaus möglich, ohne dass in diesem Zeitraum Reinigungen dieser Anlagenteile erforderlich wären.

Die Erfindung betrifft ferner ein Verfahren, bei dem die Hochsiederkolonne ohne Unterbrechung zwischen sechs und vierundzwanzig Monaten betrieben wird.

Die Erfindung betrifft auch eine Vorrichtung zur Herstellung von Vinylchlorid durch thermische Spaltung von 1,2- Dichlorethan in einem Vinylchlorid-Komplex mit einer darin enthaltenen destillativen Reinigung von 1,2-Dichlorethan und einer gegebenenfalls daran angeschlossenen Polyvinylchlorid-Teilanlage umfassend die Elemente
A) mindestens eine Hochsiederkolonne in der Teilanlage zur destillativen Reinigung von 1,2-Dichlorethan, in der höher als 1,2-Dichlorethan siedende Substanzen abgetrennt werden,
B) mindestens einen mit der Hochsiederkolonne verbundener Wärmetauscher, in den mindestens ein Teil des Kopfstroms der Hochsiederkolonne verbracht wird, dort unter Gewinnung von Wärme durch Erzeugung von Niederdruck-Wasserdampf kondensiert und gegebenenfalls unterkühlt wird, und sodann in die Hochsiederkolonne rückgeführt wird, und
C) mindestens eine Wärmesenke in einer Teilanlage zur Herstellung von 1,2-Dichlorethan angeschlossenen Teilanlage
   - zur Herstellung von Vinylchlorid und/oder
   - zur Herstellung von Polyvinylchlorid,
      in welche der im Wärmetauscher B) erzeugte Niederdruck-Wasserdampf zum Beheizen verbracht wird,
      dadurch gekennzeichnet, dass
   - die Wärmesenke sich in einer Teilanlage des Vinylchlorid-Komplexes befindet und eine Strippkolonne zur Reinigung oder Entfernung von Chlorwasserstoff aus dem Vinylchlorid ist; und/oder
   - die Wärmesenke sich in einer Polyvinylchlorid-Teilanlage befindet und eine Vorrichtung zur Entfernung von Rest-Monomer aus PVC, eine Vorentgasungseinrichtung und eine nachgeschaltete Entgasungskolonne, eine Strippkolonne zur Entfernung von Vinylchloridmonomer aus Abwasser, eine Vorrichtung zur Trocknung von PVC-Pulver und/oder eine Vorrichtung zur Erwärmung von Chargierwasser für die Polymerisationsreaktion ist.

Als Wärmesenken in den Anlagenteilen des VCM-Komplexes bzw. der PVC-Teilanlage werden bevorzugt die weiter oben beschriebenen Vorrichtungen genutzt.

Das erfindungsgemäße Verfahren bzw. die erfindungsgemäße Vorrichtung gestattet eine deutliche Verbesserung der Energiebilanz des Anlagenkomplexes.

## Patentansprüche

1. Verfahren zur Herstellung von Vinylchlorid durch thermische Spaltung von 1,2-Dichlorethan in einem Vinylchlorid-Komplex mit einer darin enthaltenen destillativen Reinigung von 1,2-Dichlorethan umfassend mindestens eine Hochsiederkolonne, in der höher als 1,2-Dichlorethan siedende Substanzen abgetrennt werden und einer gegebenenfalls daran angeschlossenen Polyvinylchlorid-Teilanlage mit den Maßnahmen
a) Betrieb der Hochsiederkolonne bei Kopftemperaturen zwischen 120 - 150 °C, und
b) Verwendung zumindestens eines Teils des Kopfstromes der Hochsiederkolonne zur Gewinnung von thermischer Energie, die in Wärmesenken einer nachgeschalteten Teilanlage zur Erzeugung von Vinylchlorid und/oder in Wärmesenken einer nachgeschalteten Teilanlage zur Herstellung von Polyvinylchlorid genutzt wird, wobei
c) der Kopfstrom zu einer indirekten Beheizung von Wärmesenken verwendet wird, indem zumindest ein Teil des Kopfstromes der Hochsiederkolonne in einem Wärmetauscher zur Erzeugung von Niederdruck-Wasserdampf genutzt wird und der Kopfstrom nach Kondensation und gegebenenfalls Unterkühlung in die Hochsiederkolonne rückgeführt wird und der Niederdruck-Wasserdampf zum Beheizen von ausgewählten Anlagenteilen eingesetzt wird,
**dadurch gekennzeichnet, dass** die Anlagenteile ausgewählt sind aus
- folgendem Anlagenteil aus dem Vinylchlorid-Komplex: einer Strippkolonne zur Reinigung oder Entfernung von Chlorwasserstoff aus dem Vinylchlorid; und/oder
- folgenden Anlagenteilen aus der Polyvinylchlorid-Teilanlage: Vorrichtungen zur Entfernung von Rest-Monomer aus PVC, einer Vorentgasungseinrichtung und einer nachgeschalteten Entgasungskolonne, einer Strippkolonne zur Entfernung von Vinylchloridmonomer aus Abwasser, einer Vorrichtung zur Trocknung von PVC-Pulver und/oder einer Vorrichtung zur Erwärmung von Chargierwasser für die Polymerisationsreaktion.

2. Verfahren nach Anspruch 1, **dadurch gekennzeichnet, dass** für die indirekte Beheizung von Wärmesenken Wärmetauscher eingesetzt werden, die ausgewählt sind aus der Gruppe der Fallstromverdampfer, Plattenwärmetauscher, Spiralwärmetauscher oder Rohrbündelwärmetauscher, wobei letztere mit speziell für einen Wärmetausch bei niedrigen Temperaturdifferenzen geeigneten Rohren ausgerüstet sind.

3. Verfahren nach Anspruch 1, **dadurch gekennzeichnet, dass** bei der Herstellung von Vinylchlorid und Polyvinylchlorid das Sumpfprodukt der Hochsiederkolonne einen DCE-Anteil von 90 - 97 Gew. % aufweist.

4. Verfahren nach Anspruch 1, **dadurch gekennzeichnet, dass** das destillativ in der Hochsiederkolonne gereingte DCE ohne weitere Behandlung zur thermischen Spaltung zu Vinylchlorid eingesetzt wird.

5. Verfahren nach Anspruch 1, **dadurch gekennzeichnet, dass** die Hochsiederkolonne ohne Unterbrechung zwischen sechs und vierundzwanzig Monaten betrieben wird.

6. Vorrichtung zur Herstellung von Vinylchlorid durch thermische Spaltung von 1,2-Dichlorethan in einem Vinylchlorid-Komplex mit einer darin enthaltenen destillativen Reinigung von 1,2-Dichlorethan und einer gegebenenfalls daran angeschlossenen Polyvinylchlorid-Teilanlage umfassend die Elemente
A) mindestens eine Hochsiederkolonne in der Teilanlage zur destillativen Reinigung von 1,2-Dichlorethan, in der höher als 1,2-Dichlorethan siedende Substanzen abgetrennt werden,
B) mindestens einen mit der Hochsiederkolonne verbundener Wärmetauscher, in den mindestens ein Teil des Kopfstroms der Hochsiederkolonne verbracht wird, dort unter Gewinnung von Wärme durch Erzeugung von Niederdruck-Wasserdampf kondensiert und gegebenenfalls unterkühlt wird, und sodann in die Hochsiederkolonne rückgeführt wird, und
C) mindestens eine Wärmesenke in einer Teilanlage zur Herstellung von 1,2-Dichlorethan angeschlossenen Teilanlage
- zur Herstellung von Vinylchlorid und/oder
- zur Herstellung von Polyvinylchlorid,
in welche der im Wärmetauscher B) erzeugte Niederdruck-Wasserdampf zum Beheizen verbracht wird,
**dadurch gekennzeichnet, dass**
- die Wärmesenke sich in einer Teilanlage des Vinylchlorid-Komplexes befindet und eine Strippkolonne zur Reinigung oder Entfernung von Chlorwasserstoff aus dem Vinylchlorid ist; und/oder
- die Wärmesenke sich in einer Polyvinylchlorid-Teilanlage befindet und eine Vorrichtung zur Entfernung von Rest-Monomer aus PVC, eine Vorentgasungseinrichtung und eine nachgeschaltete Entgasungskolonne, eine Strippkolonne zur Entfernung von Vinylchloridmonomer aus Abwasser, eine Vorrichtung zur Trocknung von PVC-Pulver und/oder eine Vorrichtung zur Erwärmung von Chargierwasser für die Polymerisationsreaktion ist.

7. Vorrichtung nach Anspruch 6, **dadurch gekennzeichnet, dass** der Wärmetauscher ausgewählt wird aus der Gruppe der Fallstromverdampfer, Plattenwärmetauscher, Spiralwärmetauscher oder Rohrbündelwärmetauscher, wobei letztere mit speziell für einen Wärmetausch bei niedrigen Temperaturdifferenzen geeigneten Rohren ausgerüstet sind.

## Claims

1. Process for production of vinyl chloride by thermal cleavage of 1,2-dichloroethane in a vinyl chloride complex incorporating a distillative purification of 1,2-dichloroethane comprising at least one high-boilers column in which substances boiling higher than 1,2-dichloroethane are removed and incorporating an optionally attached polyvinyl chloride plant, said process involving the measures of
a) operating the high-boilers column at overhead temperatures between 120-150°C, and
b) using at least part of the overhead stream from the high-boilers column to obtain thermal energy used in heatsinks of a downstream plant component dedicated to producing vinyl chloride, and/or in heatsinks of a downstream plant component dedicated to producing polyvinyl chloride, with
c) the overhead stream being used for indirect heating of heatsinks by using at least part of the overhead stream from the high-boilers column in a heat exchanger to generate low-pressure steam and returning the overhead stream into the high-boilers column following condensation with or without supercooling and using the low-pressure steam for heating selected parts of the plant, **characterized in that** the parts of the plant are selected from
- the following plant component from the vinyl chloride complex: a stripping column for purifying or removing hydrogen chloride from the vinyl chloride; and/or
- the following plant components from the polyvinyl chloride plant: apparatuses for removing residual monomer from PVC, a predevolatilizing device and a downstream devolatilizing column, a stripping column for removing vinyl chloride monomer from wastewater, an apparatus for drying PVC powder and/or an apparatus for heating batch water for the polymerization reaction.

2. Process according to Claim 1 **characterized in that** the indirect heating of heatsinks is effected using heat exchangers selected from the group of falling-stream evaporators, plate-type heat exchangers, coil-type heat exchangers or tube-bundle heat exchangers, the latter being fitted with tubes specifically suitable for heat exchange at low temperature differences.

3. Process according to Claim 1 **characterized in that** the bottom product from the high-boilers column has a DCE content of 90-97 wt% in the production of vinyl chloride and polyvinyl chloride.

4. Process according to Claim 1, **characterized in that** the DCE which has been purified by distillation in the high-boilers column is used without further treatment for the thermal dissociation to form vinyl chloride.

5. Process according to Claim 1, **characterized in that** the high-boilers column is operated without interruption for from six to twenty-four months.

6. Apparatus for production of vinyl chloride by thermal cleavage of 1,2-dichloroethane in a vinyl chloride complex incorporating a distillative purification of 1,2-dichloroethane and an optionally attached polyvinyl chloride plant, said apparatus comprising the elements
A) at least one high-boilers column in the plant component dedicated to the distillative purification of 1,2-dichloroethane where substances boiling higher than 1,2-dichloroethane are removed,
B) at least one heat exchanger which is connected to the high-boilers column and into which at least part of the overhead stream from the high-boilers column is conveyed to be condensed and optionally supercooled therein to obtain heat by generating low-pressure steam and then to be returned into the high-boilers column, and
C) at least one heatsink in a component plant attached to the component plant for production of 1,2-dichloroethane
- for production of vinyl chloride and/or
- for production of polyvinyl chloride,
into which the low-pressure steam generated in heat exchanger B) is conveyed for heating purposes, **characterized in that**
- the heatsink is situated in a component plant of the vinyl chloride complex and is a stripping column for purifying or removing hydrogen chloride from the vinyl chloride; and/or
- the heatsink is situated in a polyvinyl chloride plant and is an apparatus for removing residual monomer from PVC, a predevolatilizing device and a downstream devolatilizing column, a stripping column for removing vinyl chloride monomer from wastewater, an apparatus for drying PVC powder and/or an apparatus for heating batch water for the polymerization reaction.

7. Apparatus according to Claim 6 **characterized in that** the heat exchanger is selected from the group of falling-stream evaporators, plate-type heat exchangers, coil-type heat exchangers or tube-bundle heat exchangers, the latter being fitted with tubes specifically suitable for heat exchange at low temperature differences.

## Revendications

1. Procédé pour la préparation de chlorure de vinyle par dissociation thermique de 1,2-dichloroéthane dans un complexe de production de chlorure de vinyle incluant une purification de 1,2-dichloroéthane par distillation comprenant au moins une colonne pour substances lourdes, dans laquelle les substances ayant un point d'ébullition supérieur à celui du 1,2-dicholoroéthane sont séparées et une installation partielle de production de poly(chlorure de vinyle) qui y est éventuellement associée, pourvu des actions de
a) exploitation de la colonne pour substances lourdes à des températures de tête de 120-150°C et
b) utilisation d'au moins une partie du flux de tête de la colonne pour substances lourdes pour la récupération d'énergie thermique, qui est utilisée dans les dissipateurs thermiques d'une installation partielle de production de chlorure de vinyle située en aval et/ou dans les dissipateurs thermiques d'une installation partielle de production de poly(chlorure de vinyle) située en aval,
c) le flux de tête étant utilisé pour un chauffage indirect de dissipateurs thermiques, en ce qu'au moins une partie du flux de tête de la colonne pour substances lourdes est utilisée dans un échangeur thermique pour la production de vapeur d'eau à basse pression et le flux de tête, après condensation et éventuellement sous-refroidissement, est reconduit dans la colonne pour substances lourdes et la vapeur d'eau à basse pression est utilisée pour le chauffage de parties d'installations choisies, **caractérisé en ce que** les parties d'installations sont choisies parmi
- les parties d'installations suivantes du complexe de production de chlorure de vinyle : une colonne de rectification pour la purification ou l'élimination de chlorure d'hydrogène du chlorure de vinyle ; et/ou
- les parties d'installations suivantes du complexe de production de poly(chlorure de vinyle) : des dispositifs pour l'élimination de restes de monomères du PVC (poly(chlorure de vinyle)), un dispositif de purge préalable et une colonne de purge en aval, une colonne de rectification pour l'élimination de monomères de chlorure de vinyle des eaux usées, un dispositif pour le séchage de poudre de PVC et/ou un dispositif pour le réchauffage d'eau de charge pour la réaction de polymérisation.

2. Procédé selon la revendication 1, **caractérisé en ce que** des échangeurs thermiques sont employés pour le chauffage indirect de dissipateurs thermiques, choisis dans le groupe des évaporateurs à flux descendant, des échangeurs thermiques à plaques, des échangeurs thermiques en spirale et des échangeurs thermiques à faisceau tubulaire, ces derniers étant équipés de tubes spécialement appropriés pour un échange thermique à des faibles différences de température.

3. Procédé selon la revendication 1, **caractérisé en ce que** lors de la préparation de chlorure de vinyle et de poly(chlorure de vinyle), le produit de fond de la colonne pour substances lourdes présente une proportion de 90-97% en poids de DCE (1,2-dichloroéthane).

4. Procédé selon la revendication 1, **caractérisé en ce que** le DCE purifié par distillation dans la colonne pour substances lourdes est utilisé sans manipulation supplémentaire pour la dissociation thermique en chlorure de vinyle.

5. Procédé selon la revendication 1, **caractérisé en ce que** la colonne pour substances lourdes fonctionne sans interruption pendant six à vingt-quatre mois.

6. Dispositif de préparation de chlorure de vinyle par dissociation thermique de 1,2-dichloroéthane dans un complexe de production de chlorure de vinyle incluant une purification de 1,2-dichloroéthane par distillation et une installation partielle de production de poly(chlorure de vinyle) qui y est éventuellement associée, comprenant les éléments de
A) au moins une colonne pour substances lourdes dans l'installation partielle pour la purification par distillation de 1,2-dichloroéthane, dans laquelle les substances ayant un point d'ébullition supérieur à celui du 1,2-dichloroéthane sont séparées,
B) au moins un échangeur thermique relié à la colonne pour substances lourdes, dans lequel au moins une partie du flux de tête de la colonne pour substances lourdes est acheminée, se condense là avec formation de chaleur par la production de vapeur d'eau à basse pression et éventuellement sous-refroidie et est ensuite reconduite dans la colonne pour substances lourdes et
C) au moins un dissipateur thermique d'une installation partielle associée à l'installation partielle de préparation de 1,2-dichloroéthane
- pour la préparation de chlorure de vinyle et/ou
- pour la préparation de poly(chlorure de vinyle),
dans laquelle la vapeur d'eau à basse pression produite dans l'échangeur thermique B) est acheminée pour le chauffage,
**caractérisé en ce que**
- le dissipateur thermique se trouve dans une installation partielle du complexe de production de chlorure de vinyle et est une colonne de rectification pour la purification ou l'élimination de chlorure d'hydrogène du chlorure de vinyle ; et/ou
- le dissipateur thermique se trouve dans une installation partielle de production de poly(chlorure de vinyle) et est un dispositif pour l'élimination de restes de monomères du PVC, un dispositif de purge préalable et une colonne de purge en aval, une colonne de rectification pour l'élimination de monomères de chlorure de vinyle des eaux usées, un dispositif pour le séchage de poudre de PVC et/ou un dispositif pour le réchauffage d'eau de charge pour la réaction de polymérisation.

7. Dispositif selon la revendication 6, **caractérisé en ce que** l'échangeur est choisi dans le groupe des évaporateurs à flux descendant, des échangeurs thermiques à plaques, des échangeurs en spirale et des échangeurs thermiques à faisceau tubulaire, ces derniers étant équipés de tubes spécialement appropriés pour un échange thermique à des faibles différences de température.
